# DEMANDE DE BREVET EUROPEEN

(11) **EP 0 709 710 A1**
(43) Date de publication de la demande: **01.05.1996**
(21) Numéro de dépôt: 95402353.7
(22) Date de dépôt: 23.10.1995
(51) Int. Cl.: G02F 1/01, G02B 6/13

(54) **Modulateur électro-optique à mode tranverse électrique et procédé de réalisation d'un tel modulateur**

(30) Priorité: 25.10.1994 FR 9412736
(71) Demandeur: COMMISSARIAT A L'ENERGIE ATOMIQUE, F-75015 Paris (FR)
(72) Inventeur: Faderl, Ingo, F-63000 Clermont-Ferrand (FR); Valette, Serge, F-38100 Grenoble (FR)
(74) Mandataire: Dubois-Chabert, Guy

(57) **Abrégé**

L'invention concerne une cellule électro-optique à mode transverse électrique pour un modulateur, et son procédé de réalisation.

La cellule comporte sur un substrat (4), une région active (10), et des électrodes de commande (16) de modulation disposées de part et d'autre de la région active (10). Selon l'invention, la cellule comporte des régions de confinement (14) en un matériau de plus faible indice de réfraction et de plus faible résistivité que le matériau de la région active, les régions de confinement étant disposées de part et d'autre et contre la région active (10) et entre les électrodes de commande (16).

## Description

### Domaine technique

La présente invention concerne une cellule électro-optique à mode transverse électrique et son procédé de réalisation. Elle concerne plus particulièrement des cellules électro-optiques avec des matériaux polymères non linéaires, des matériaux hybrides non linéaires ou des cristaux liquides. Ces cellules sont utilisées dans des modulateurs électro-optiques. On entend par cellule électro-optique à mode transverse électrique une cellule électro-optique apte à fonctionner de manière optimale dans la polarisation de champ électromagnétique dite transverse électrique (champ électrique perpendiculaire au plan d'incidence défini par la direction de propagation et la normale au plan de propagation) . L'invention trouve donc de nombreuses applications dans le domaine du traitement des signaux, des télécommunications par voie optique ou encore dans la réalisation de capteurs, d'analyseurs de spectres et de dispositifs d'interférométrie. Une application particulière est, par exemple, la réalisation d'un interféromètre de type Mach-Zehnder.

### Etat de la technique antérieure

On rappelle qu'une structure guide d'ondes optiques consiste généralement en une couche tampon, une couche active et une couche supérieure empilées sur un substrat, la couche active ayant un indice de réfraction supérieur à celui des couches tampon et supérieure.

L'effet électro-optique permet de modifier, sous l'action d'un champ électrique, l'indice de réfraction d'un matériau et donc de réaliser des fonctions diverses comme la modulation de phase ou d'intensité d'un signal lumineux, ou encore le changement de polarisation de ce signal. Le champ électrique est appliqué au matériau actif par l'intermédiaire d'électrodes désignées par "électrodes de commande" dans la suite du texte.

On peut se reporter à ce sujet, par exemple, au documents 1 et 2 référencés à la fin de la présente description.

Pour fabriquer une cellule pour modulateur électro-optique, la couche active comporte, par exemple, des polymères non linéaires tels que commercialisés sous les désignations DR1-PMMA (commercialisé par la Société IBM aux Etats-Unis) ou PU11 (commercialisé par Flamel Technology France).

Pour que ces polymères présentent un effet de deuxième ordre, en l'occurrence la modification de l'indice de réfraction, il faut que tous les chromophores de ces polymères soient orientés dans une même direction. Cette direction dépend de l'emplacement des électrodes permettant d'appliquer le champ électrique et du mode de fonctionnement. Pour avoir un coefficient électro-optique le plus élevé, un fonctionnement en mode transverse magnétique (TM) implique que les électrodes de commande sont disposées en-dessus et en-dessous de la couche active et un fonctionnement en mode transverse électrique (TE) implique que les électrodes sont disposées de deux côtés de la couche active. L'orientation du polymère par rapport à la disposition des électrodes est, par exemple, décrit dans le document 3 référencé à la fin de la description.

L'orientation des polymères est réalisée en chauffant ceux-ci à leur température vitreuse et en appliquant simultanément un fort champ électrique, dit "champ de polarisation". Le polymère est ensuite refroidi tout en maintenant le champ, ce qui permet de conserver l'orientation lorsque le polymère est froid.

Pour exploiter la plus grande non-linéarité du polymère, il faut que le champ électrique de polarisation, le champ électrique de modulation appliqué à la couche active et le champ électrique de l'onde optique soient tous trois dirigés dans la même direction. Ceci explique les différences de configuration des électrodes pour les modes de fonctionnement en TE et TM.

Par définition, on entend par structure guidante, l'ensemble des couches permettant le confinement de la lumière. Généralement une structure guidante comporte un coeur intercalé entre des couches de matériaux d'indices de réfraction inférieurs à celui du coeur. Pour assurer un confinement latéral de la lumière dans la structure, le coeur et/ou la couche de matériau située au-dessus du coeur peuvent avoir au moins une partie de largeur limitée. Le confinement latéral peut être également obtenu par la gravure d'une rainure dans la couche de matériau située sous le coeur de façon à ce qu'au moins une partie du matériau formant le coeur soit déposée dans cette rainure. Dans le cas d'un modulateur électro-optique, le coeur de la structure que l'on appelle par la suite "guide optique" (car il assure la propagation de la lumière) comporte un matériau actif. Lorsque ce matériau actif est un polymère, le confinement latéral peut être aussi obtenu directement par une modification locale du matériau actif sous l'effet d'une insolation de ce dernier par un rayonnement ultraviolet.

On peut se reporter à ce sujet, par exemple, aux documents 1, 2 ou 3 référencés à la fin de la présente description.

Les cellules électro-optiques connues fonctionnant selon le mode transverse électrique (TE) présentent un certain nombre d'inconvénients et de limitations tant dans leur fonctionnement que dans leur réalisation industrielle.

L'une des limitations provient de la tension de commande très élevée nécessaire à l'obtention de l'effet électro-optique non linéaire. Cette tension élevée est due notamment à l'éloignement des électrodes de commande qui créent un champ électrique dans le matériau actif. A ce sujet, il faut toutefois noter qu'il est impossible de rapprocher les électrodes en raison de la forme et de l'étendue du faisceau optique dans le matériau et donc impossible de réduire la tension de commande.

Un autre problème tient à la tension de polarisation très élevée qui est nécessaire pour appliquer le champ électrique lors de l'opération d'orientation du matériau actif, en l'occurrence du polymère. Comme le mentionne le document 1, par exemple, la tension de polarisation est susceptible de détruire certaines parties du modulateur si des précautions particulières ne sont pas prises.

Un autre inconvénient d'un grand nombre de matériaux actifs, et en particulier de polymères non linéaires, est celui de leur très grande fragilité. Les polymères présentant des efficacités intéressantes dans leurs propriétés électro-optiques ne sont en général pas utilisables dans les procédés courants de fabrication industrielle. Par exemple, nombre de polymères non linéaires supportent difficilement les rayonnements ultraviolets. Ceux-ci cassent leur colorant et détruisent par conséquent l'effet non linéaire. Or, l'usage des rayonnements ultraviolets est fréquent dans les procédés de dépôt et de gravure de la silice, par exemple.

La fragilité des polymères se traduit aussi par leur mauvaise résistance à l'échauffement. Beaucoup de polymères ne supportent effectivement pas un échauffement à une température supérieure à 150°C. Ceci les rend ainsi inutilisables dans la réalisation de dispositifs mettant en jeu des opérations exécutées à des températures élevées, telles que les opérations de recuit.

D'autres opérations de fabrication des composants sont également incompatibles avec l'usage de polymères. Les polymères non linéaires ne peuvent pas être étalés en couches minces sur un substrat ou une structure présentant des motifs ou de fortes inhomogénéités de surface telles que, par exemple, des électrodes épaisses.

Un inconvénient supplémentaire d'un grand nombre de polymères non linéaires est leur nature hygroscopique. Après la préparation de la couche de polymère, celle-ci doit être polarisée, comme cela est décrit ci-dessus, pour permettre un effet électro-optique. L'eau qui entre par la suite dans le polymère ramollit en général sa structure et fait disparaître l'ordre induit par la polarisation de la couche. Par conséquent, l'effet non linéaire disparaît au fur et à mesure que la couche de polymère "prend l'eau".

La présente invention a pour objet de proposer un modulateur électro-optique, ainsi que son procédé de fabrication, nécessitant une tension de polarisation et une tension de commande réduites, et ne présentant pas les inconvénients évoqués ci-dessus. Un autre objet est de proposer un procédé de fabrication qui évite toute altération du matériau actif, et qui soit d'une mise en oeuvre aisée.

### Exposé de l'invention

A cet effet, l'invention propose un modulateur électro-optique à mode transverse électrique comportant au moins un guide optique avec une région active en un matériau non-linéaire ayant un indice de réfraction pouvant être modifié sous l'application d'un champ électrique au matériau, et des électrodes de commande pour l'application du champ électrique disposées de part et d'autre du guide, caractérisé en ce qu'il comporte en outre des régions en un matériau de confinement disposées respectivement de part et d'autre et contre la région active, et entre les électrodes de commande, le matériau de confinement présentant un indice de réfraction inférieur à celui du matériau de la région active, et une résistivité électrique inférieure à celle du matériau de la région active.

Grâce à l'invention et en particulier à la présence des régions en matériau de confinement avec un faible indice de réfraction, la largeur du mode dans le guide d'onde est réduite, il est donc possible de rapprocher les électrodes de commande l'une de l'autre sans provoquer de pertes notables de l'onde optique confinée dans le matériau actif. Pour une cellule électro-optique, la tension de commande est directement proportionnelle à l'espacement entre électrodes ; il est donc possible de réduire cette tension. Les régions de confinement permettent de réduire la tension de commande typiquement de 30 à 40%. Grâce aux régions de confinement, il est possible également de réduire la tension de polarisation du matériau actif et en particulier du polymère non linéaire lors de son orientation. Les régions de confinement permettent en effet de limiter le champ de polarisation à la région active.

Le modulateur électro-optique de l'invention présente une structure permettant de répondre à la double exigence d'un bon confinement de la lumière, avec une propagation de celle-ci sans pertes notables, et d'un confinement du champ électrique le meilleur possible au sein du volume occupé par le matériau de la région active.

L'utilisation d'un matériau de confinement avec un indice de réfraction dont la partie réelle est plus faible que celle du matériau de la région active assure le bon confinement de la lumière. De plus, un indice dont la partie imaginaire est faible permet de limiter les pertes de propagation à des valeurs négligeables.

Comme le matériau de confinement présente en outre une résistivité plus faible que le matériau actif, un bon confinement électrique est possible sans modifier beaucoup le coefficient d'extinction optique. A titre indicatif le matériau actif peut présenter une résistivité de l'ordre de 10¹³ Ω.cm, ou plus.

On peut noter que, pour améliorer le confinement électrique, l'idée la plus simple serait, à titre de comparaison, de placer les électrodes de commande à proximité immédiate du matériau de la région active. Les électrodes, généralement en métal, présentent effectivement une résistivité très faible de l'ordre de 10⁻⁶ Ω.cm. Toutefois, le matériau métallique des électrodes possède un indice de réfraction dont la partie imaginaire est très élevée et conduisant donc à des pertes de propagation lumineuse prohibitives.

De façon avantageuse le matériau de confinement du dispositif de l'invention peut être choisi avec une résistivité comprise entre 10⁵ et 10¹⁰ Ω.cm, par exemple.

On considère dans une application pratique qu'une absorption de la lumière de l'ordre de 10⁻¹⁰ cm⁻¹ à 10⁻⁴ cm⁻¹ peut être considérée comme négligeable.

Selon un aspect avantageux de l'invention, le matériau de la région active peut être un polymère non linéaire.

Selon un autre aspect de l'invention, la cellule peut comporter également une couche de recouvrement de la région active en un matériau présentant un indice de réfraction inférieur à celui du matériau de la région active, et présentant une faible constante diélectrique, c'est-à-dire, de façon générale, inférieure à 4. Le matériau de cette couche est choisi pour ses caractéristiques optiques et électriques, il doit présenter un faible indice de réfraction et une faible constante diélectrique afin d'éviter tout claquage lors de la polarisation, c'est-à-dire l'orientation du matériau actif ("poling").

Le matériau de cette couche peut être par exemple de la silice dopée ou un polymère de la marque Téflon AF de Du Pont de Nemours. Le Téflon AF présente un indice optique n=1,29 et une constante diélectrique ε=1,87 à 1,93 pour une fréquence de l'ordre de 10GHz.

La cellule électro-optique peut, par ailleurs, comporter un substrat de support de la région active, des électrodes et du matériau de confinement, qui présente une partie gravée contenant partiellement la région active. La gravure du substrat assure alors avec le matériau de confinement, le confinement latéral de la lumière dans la structure.

Selon une réalisation particulière de l'invention, le guide optique comporte également un ruban en un matériau présentant un indice de réfraction supérieur à celui du matériau de la région active. Le ruban s'étend le long de la région active, et contribue au guidage de la lumière.

L'invention concerne aussi un procédé de réalisation d'une cellule pour modulateur telle que décrite ci-dessus. Ce procédé comporte pour l'essentiel les opérations suivantes :
- formation d'un substrat en un matériau isolant électrique et ayant un indice inférieur à l'indice du matériau de la couche active,
- formation sur le substrat des électrodes de commande,
- formation des régions en un matériau de confinement,
- formation d'une couche de matériau "sacrificiel" et configuration de la couche selon un motif permettant de fixer l'emplacement et la forme de la région active,
- formation d'une couche de recouvrement sur la couche de matériau sacrificiel,
- réalisation d'au moins une ouverture dans la couche de recouvrement jusqu'à la couche de matériau sacrificiel,
- élimination de la couche de matériau sacrificiel pour former une cavité correspondant à la région active et remplissage de la cavité avec un matériau non linéaire.

Grâce à ce procédé de fabrication, il est possible de traiter les plaques de circuit intégré selon des techniques traditionnelles pour la réalisation de la cellule jusqu'au moment où le matériau actif est introduit dans la cavité. L'usage de polymères non linéaires fragiles n'est ainsi plus incompatible avec la fabrication de la structure d'un modulateur. Il est possible, par exemple, de réaliser des gravures telles que les gravures RIE par ions réactifs (Reactive Ion Etching) avec du gaz CF₄ qui émet un rayonnement bleu et ultraviolet.

De même, une surface non plane pour le dépôt du matériau non-linéaire ne constitue, grâce à l'invention, plus un obstacle car ce dernier n'est plus étalé à la tournette comme dans l'art antérieur.

En particulier, le motif des électrodes de commande et les emplacements pour recevoir les fibres optiques reliées au modulateur ou à la cellule peuvent être conçus en fonction de leurs impératifs propres et non en fonction du matériau non-linéaire. On peut noter à ce sujet qu'il est également possible de relier des fibres optiques au modulateur avant le remplissage de la cavité avec le matériau actif.

Le matériau actif (ou non-linéaire) n'est en outre plus assujetti aux effets des acides, bases ou solvants. Un matériau actif présentant une bonne efficacité dans ses propriétés optiques peut donc être retenu sans tenir compte de son éventuelle fragilité.

Les électrodes de commande sont de préférence réalisées avant le remplissage de la cavité avec le matériau actif. Selon différents modes de mise en oeuvre du procédé, elles peuvent être réalisées, par exemple, directement après la formation du substrat, soit après la formation des régions en matériau de confinement à résistance électrique réduite soit encore après l'élimination du matériau sacrificiel. Des dépressions peuvent être par ailleurs gravées dans le substrat pour recevoir les électrodes de commande et/ou le matériau actif.

D'autres caractéristiques et avantages de l'invention ressortiront de la description qui va suivre, en référence aux dessins annexés, donnée purement à titre d'illustration.

### Brève description des figures

- les figures 1 à 3 sont des coupes schématiques d'un modulateur conforme à l'invention,
- la figure 4 est une coupe schématique d'un modulateur conforme à l'invention ayant deux guides d'ondes,
- les figures 5 à 8 sont des coupes schématiques illustrant des variantes de réalisation du guide d'onde d'un modulateur conforme à l'invention,
- les figures 9 et 10 sont des coupes schématiques respectivement du guide d'onde d'un modulateur conventionnel et d'un modulateur conforme à l'invention et illustrent l'extension du mode transverse électrique,
- la figure 11 est une courbe représentant pour un modulateur conforme à la figure 10 la demi-largeur du mode TE en fonction de la largeur de la région active de ce modulateur,
- les figures 12 à 18 sont des coupes schématiques illustrant les étapes successives d'un procédé de réalisation du modulateur conforme à l'invention.

### Description détaillée de modes de réalisation de l'invention

Pour des raisons de simplification, des références identiques sont utilisées pour des parties correspondantes des différentes figures décrites ci-dessous. Par ailleurs, les différentes parties des figures ne sont pas représentées à l'échelle. En outre, on prendra comme exemple de matériau actif celui des polymères non-linéaires.

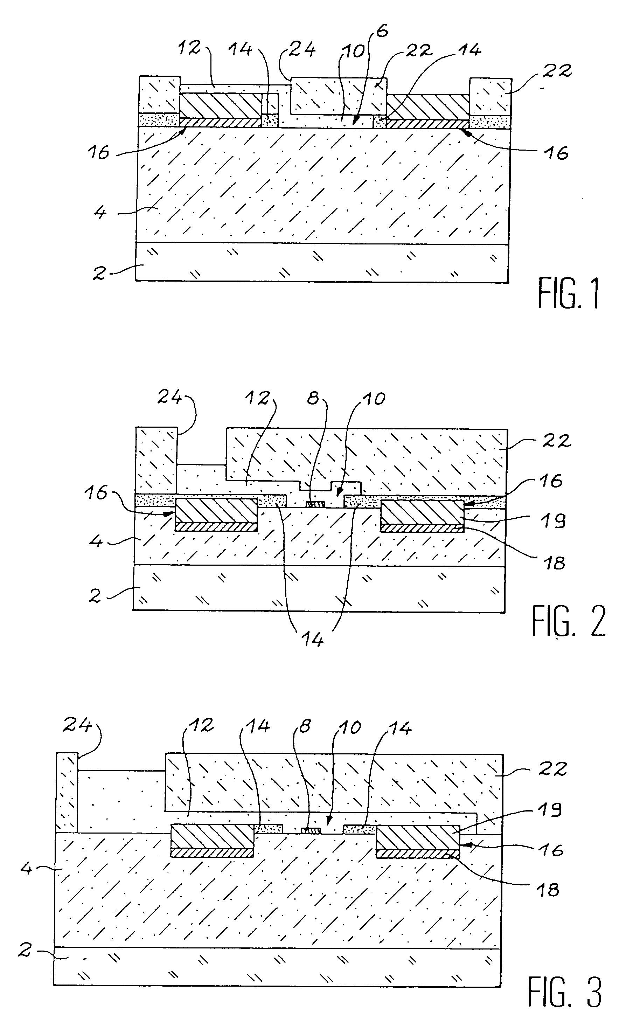

Les figures 1 à 3 montrent la structure générale de cellules pour modulateurs réalisées selon l'invention. Les cellules comportent une plaquette de silicium 2 sur laquelle est formé un substrat 4, par exemple d'oxyde de silicium dopé au phosphore et d'une épaisseur de l'ordre de 10 µm.

Un guide d'onde 6 comporte une région active 10 emplie avec un matériau actif 12 tel qu'un polymère non linéaire. Conformément à l'invention, la structure comporte également deux régions 14 en un matériau de confinement de résistance électrique inférieure à celle du matériau actif. Le matériau des régions 14 reste cependant un isolant mais avec une résistance caractéristique inférieure d'un facteur 10 à 100 à celle de la région active. Les régions 14 sont réalisées par exemple en oxyde de silicium (SiO₂) fortement dopé à l'azote ou au phosphore pour en réduire la résistance électrique. Elle sont en contact de part et d'autre et contre le matériau actif de la région 10. Il est ainsi possible de concentrer le champ électrique de la polarisation dans la région active lors de l'orientation du polymère.

Des électrodes de commande avec la référence 16 sont disposée de part et d'autre de la cavité et des régions 14 et en contact avec les régions 14. Les électrodes 16, coplanaires, peuvent être formées à la surface du substrat 4 comme le montre la figure 1, mais également dans des dépressions gravées dans le substrat. Ceci est le cas des modulateurs illustrés aux figures 2 et 3. Les électrodes 16 peuvent comporter, comme représenté sur ces figures, une couche 18 d'accrochage sur le substrat 4, qui sert essentiellement à faire croître électrolytiquement un matériau 19 qui forme le corps des électrodes 16.

Comme le montrent les figures 2 et 3, le guide d'onde 6 peut comporter également un ruban 8 de matériau d'indice de réfraction élevé, par exemple du nitrure de silicium. Le ruban 8 qui s'étend le long de la cavité contribue au guidage latéral d'un faisceau lumineux dans le matériau.

Une couche 22 recouvre la région active 10 et avantageusement les régions 14. Cette couche peut également recouvrir les électrodes 16 (figures 2 et 3). Elle peut encore être gravée pour former postérieurement les électrodes 16, qui, dans ce cas, ne sont pas recouvertes par la couche 22 (figure 1).

Une ouverture 24 pratiquée dans la couche 22 permet l'emplissage de la région active avec un polymère non-linéaire 12.

Les figures donnent des structures non symétriques, mais il peut être avantageux dans certains modes de fonctionnement tels que par exemple celui du type "push-pull" de réaliser des structures symétriques latéralement par rapport au guide optique.

Le tableau I ci-dessous donne à titre d'exemple des valeurs concernant le dimensionnement et les matériaux des différentes parties du modulateur.

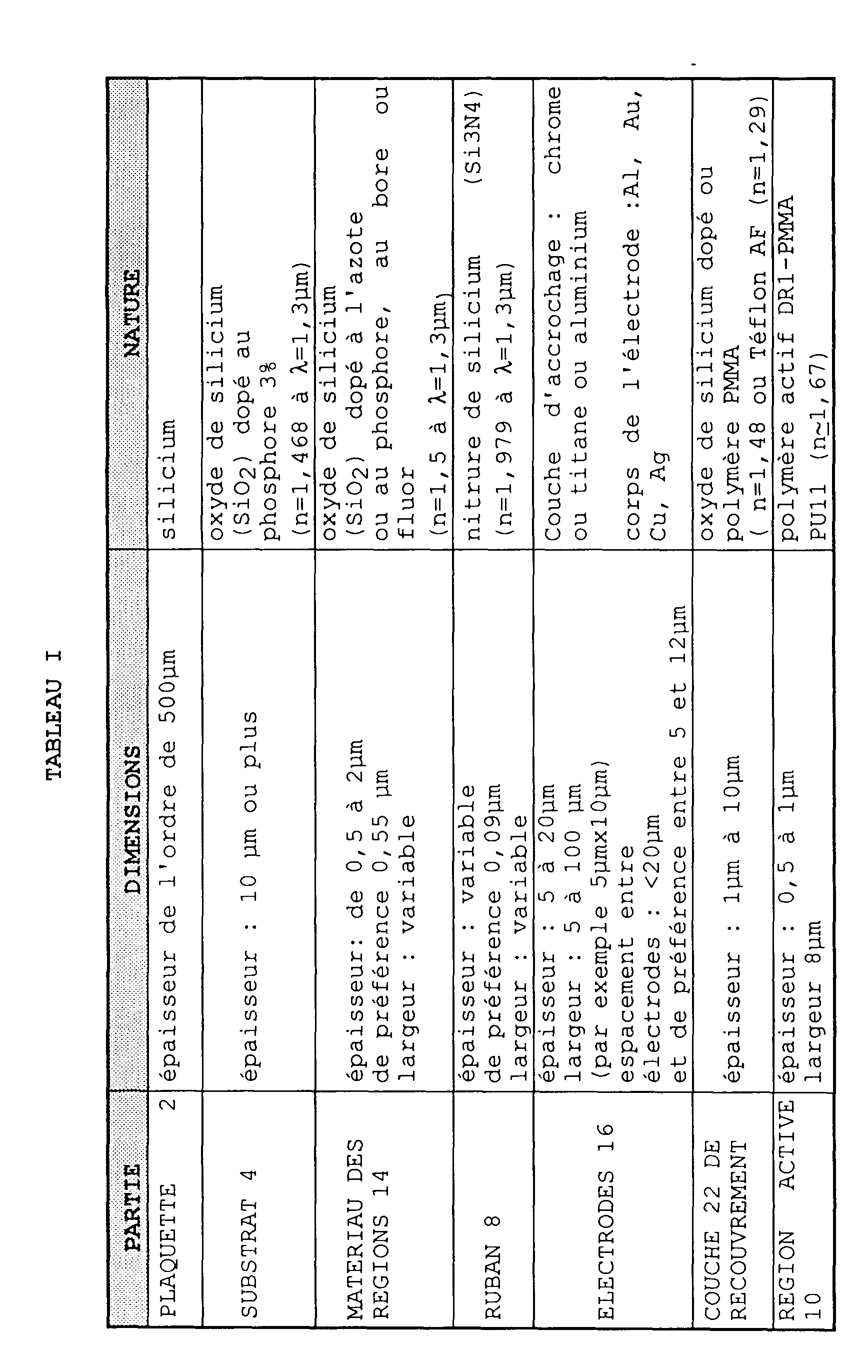

Le terme épaisseur du tableau I s'entend dans le sens de l'empilement des couches et le terme largeur s'entend parallèlement à la surface du substrat 4 dans le plan de coupe des figures.

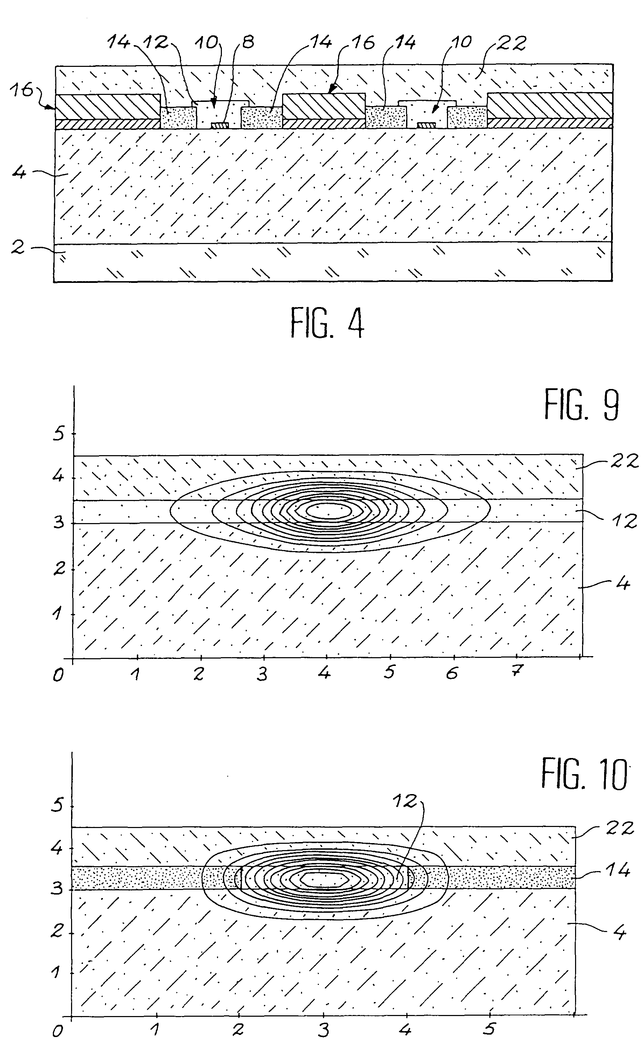

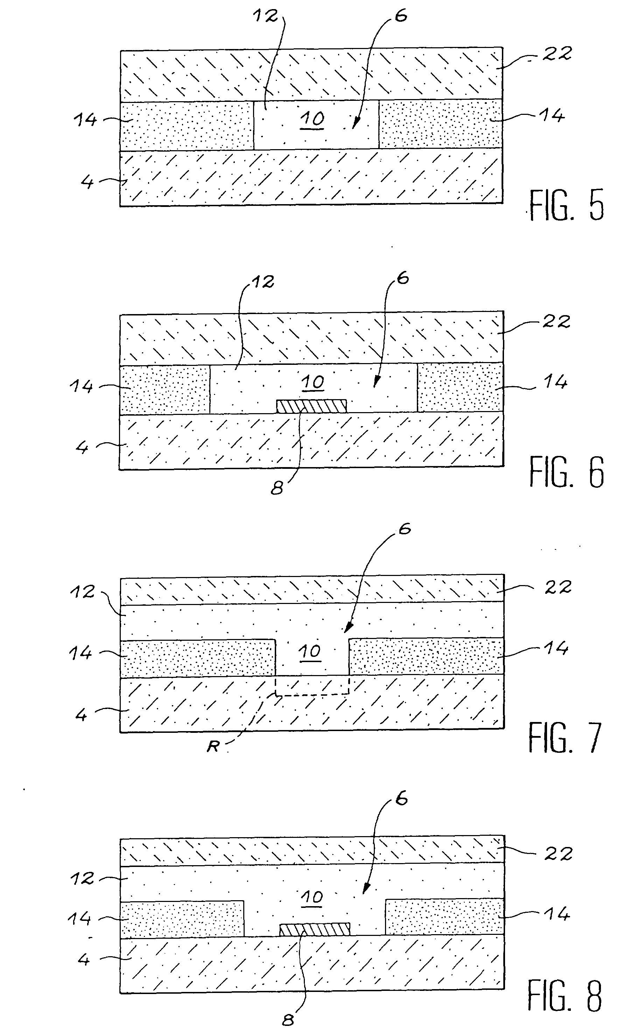

La figure 4 montre une application particulière de l'invention dans lequel la cellule est utilisée dans un modulateur de type Mach-Zehnder. La cellule comporte deux guides d'ondes 6 coplanaires s'étendant sensiblement parallèlement l'un à l'autre. Pour plus de détails concernant les interféromètres Mach-Zehnder, on peut avantageusement se reporter aux documents 2 et 4, 5 et 6 référencés à la fin de la présente description.

On retrouve sur la figure 4 des éléments correspondants aux figures 1 à 3 qui portent des références identiques et pour lesquels on peut se référer à la description des figures 1 à 3. Alors que la figure 4 peut être comprise comme une coupe d'un modulateur à deux branches actives, chacune des figures 1 à 3 peut être comprise comme une coupe d'une seule branche d'un modulateur du type Mach-Zehnder, par exemple.

On constate que le modulateur de la figure 4 comporte une électrode centrale 16 commune aux deux guides d'ondes 6. Par ailleurs, on peut noter que les régions 10 peuvent avoir une épaisseur légèrement supérieure à celle des régions 14. Cette caractéristique confère à la structure un confinement amélioré de l'onde dans les guides 6.

De façon générale, plusieurs possibilités sont envisageables pour définir le guide d'onde 6 des cellules pour modulateurs. Les figures 5 à 8 illustrent à titre d'exemple quelques unes de ces possibilités. Dans un souci de simplification des figures, celles-ci représentent uniquement le substrat 4, les régions actives 10, les régions 14 de résistance électrique réduite et la couche de recouvrement 22. Les électrodes 16 ne sont pas représentées.

La figure 5 montre un guide classique où le polymère actif 12 est situé entre deux couches 4, 22 par exemple d'oxyde de silicium. Le guidage de l'onde est assurée dans le polymère en raison de son indice plus élevé que celui des matériaux environnants 4, 14, 22.

La figure 6 montre un guide comportant un ruban 8 qui s'étend le long de la région active et qui présente un indice de réfraction plus élevé que celui du polymère actif 12.

Dans l'exemple de la figure 7, le guidage est obtenu par la gravure de la couche de matériau à résistance électrique réduite, ce qui permet en outre la définition des régions 14, et par la gravure éventuelle d'une rainure R dans le substrat, remplie par le matériau actif. On note dans ce cas que le polymère actif n'est pas uniquement situé dans la région active 10 mais s'étend au-dessus des régions 14.

Le guidage illustré à la figure 8 peut être compris comme une combinaison des solutions des figures 6 et 7. Celui-ci résulte en effet non seulement de la gravure de la couche à résistance réduite mais aussi du ruban 8 formé dans la région active 10. Cette solution est aussi la solution retenue dans les figures 2 et 3.

Dans tous les exemples de réalisation représentés aux figures 5 à 8, une rainure dans le substrat peut être utilisée.

Les figures 9 et 10 permettent de mettre en évidence les avantages procurés par les régions 14 à résistance réduite dans des cellules électro-optiques de l'invention. Ces figures comportent des axes d'abscisse et d'ordonnée graduées en µm pour faciliter leur lecture.

Dans le cas de la figure 9, une couche de matériau actif 12 se trouve entre le substrat 4 et une couche de recouvrement 22. Il n'y a toutefois pas de régions 14 pour limiter la largeur du mode optique. Dans le cas de la figure 10 un guide d'onde semblable à celui de la figure 6 est réalisé avec des régions 14 présentant une résistivité électrique réduite conformément à l'invention. Le substrat 4 est une couche de 3µm d'épaisseur de silice avec un indice n=1,468. Un ruban non représenté sur la figure pour des raisons de clarté est également prévu. Il présente une hauteur de 0,09µm, une largeur de 1,4µm et un indice n=1,979. Le polymère actif choisi est PU11 (n=1,685) avec une épaisseur de 0,5µm ; et la couche de recouvrement est en polymère PMMA (n=1,48). La largeur totale du guide qui est défini entre le matériau 14 est de 2µm. Les électrodes de commande disposées de part et d'autre du matériau 14, ne sont pas représentées pour des raisons de simplification des figures.

Il apparaît sur la figure 10 que la largeur du mode optique est réduite en raison des régions 14 à faible indice et à résistivité réduite. En comparant la demi-largeur des modes des figures 9 et 10, on constate que sur la figure 10, la demi-largeur est réduite de l'ordre de 40%. Il est ainsi possible de rapprocher les électrodes de commandes (non représentées) de 10µm à 6µm sans introduire de perte notable. La tension de commande peut ainsi être avantageusement abaissée de 40% environ.

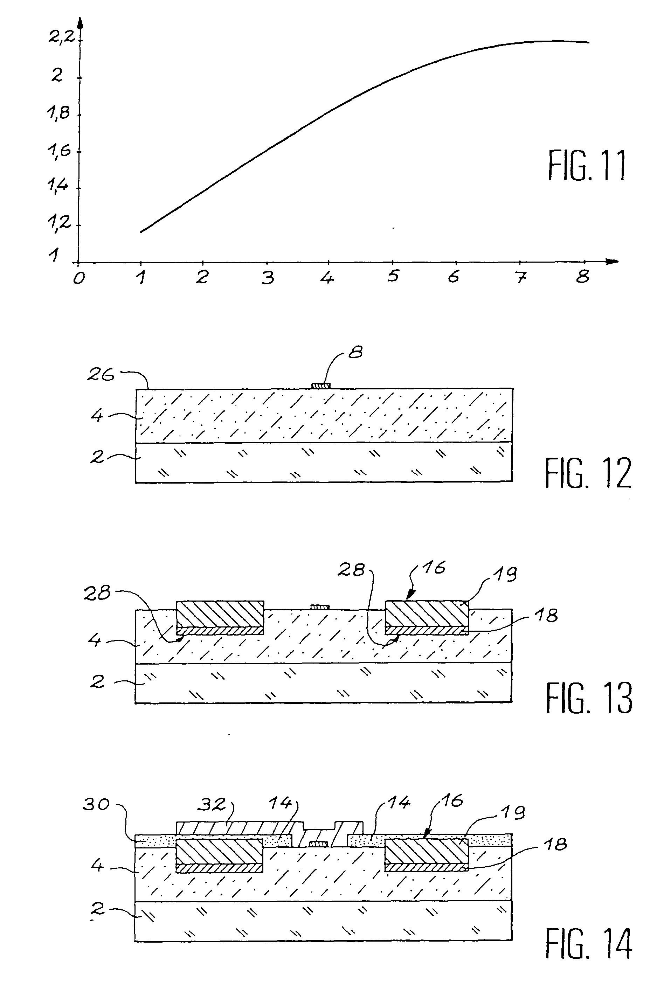

La figure 11 est une courbe représentant pour la cellule de la figure 10 la demi-largeur du mode TE, c'est-à-dire la composante électrique Ex du champ électromagnétique parallèlement au substrat et perpendiculairement au guide, en fonction de la largeur de la région active. En ordonnée, sont reportées les valeurs de Ex/10 en µm (correspondant à la demi-largeur du champ Ex prise à -10dB de la valeur maximum du champ) et en abscisse la largeur de la région active en µm. Les largeurs du mode TE sont mesurées à un endroit où l'intensité du mode est égale seulement au centième de l'intensité (correspondant à -20dB) au centre du guide. Autrement dit, l'intensité diminue au fur et à mesure qu'on s'éloigne du centre du guide. Il apparaît clairement sur la figure 11 que lorsque la largeur de la région de polymère actif diminue, le mode optique est plus confiné.

Les figures 12 à 18 illustrent à titre d'exemple une mise en oeuvre particulière du procédé de fabrication d'une cellule conformément à l'invention.

Comme le montre la figure 12, le procédé de fabrication comporte tout d'abord le dépôt d'une couche 4 par exemple de silice, sur une plaquette 2. Après le dépôt, la surface 26 de la couche 4 est lissée. Une fine couche (0,09µm) de nitrure de silicium est déposée sur la surface 26, puis gravée, par exemple par plasma d'ions réactifs RIE ("Reactive Ion Etching") pour définir le ruban 8.

Le procédé se poursuit par la gravure de dépressions 28 dans la surface 26 (figure 13) et la formation des électrodes de commande 16 dans ces dépressions. La gravure des dépressions peut être une gravure chimique humide (exemple HF+HNF₄) ou une gravure sèche par plasma de type RIE avec un gaz CHF₃ par exemple. Les électrodes 16 sont formées en deux étapes, une couche d'accrochage 18 est tout d'abord réalisée dans les dépressions 28 puis un matériau 19 est formé par électrolyse sur la couche 18.

Après la formation des électrodes 16, l'ensemble de la structure peut être rendue plane ; cette opération n'est pas représentée sur les figures.

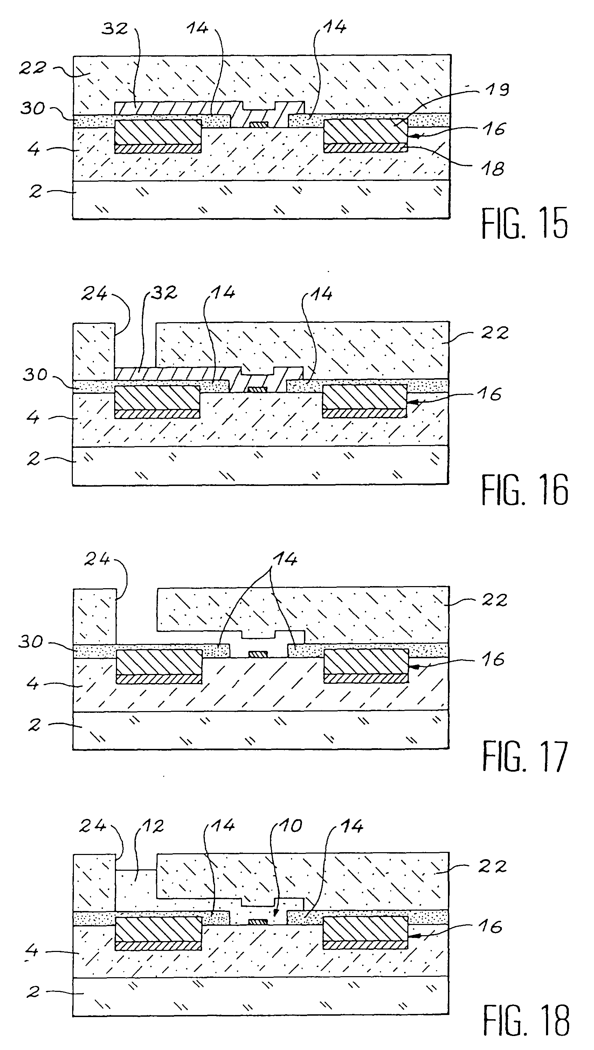

La figure 14 montre la suite du procédé qui comporte le dépôt d'une couche 30 d'un matériau ayant un indice de réfraction et une résistivité inférieure au matériau actif. La couche 30, en silice dopée à l'azote ou au phosphore ou encore au bore ou au fluor est gravée pour former les régions 14 conformément à l'invention. Une couche de matériau sacrificiel 32, par exemple en aluminium, et également déposée puis configurée selon un motif permettant de fixer la forme et l'emplacement non seulement de la région active mais plus généralement de la partie contenant le matériau actif. Le motif peut être obtenu par gravure ou par pelage (lift-off). Les couches 30, 32 et l'ensemble de la structure sont ensuite recouverts par une couche 22 de silice par exemple (figure 15).

Une ouverture 24, visible sur la figure 16, est ensuite pratiquée dans la couche 22. L'ouverture 24 est gravée jusque sur le matériau sacrificiel 32. Un traitement avec un agent convenablement choisi, en l'occurrence de l'acide sulfurique, permet d'éliminer le matériau sacrificiel 32 et de libérer ainsi une cavité qui présente le motif de la future région active. Un rinçage à l'eau et un recuit en atmosphère sèche permet en outre d'enlever toutes les substances restant dans la cavité. Cette cavité est représentée à la figure 17. La cavité est, comme le montre la figure 18, finalement remplie avec un polymère actif non-linéaire 12. Le polymère est injecté dans la cavité, mais un effet de capillarité contribue à son remplissage correct. L'injection du polymère peut être effectué en présence de vapeur de solvant du polymère pour empêcher une décomposition du polymère injecté et de son solvant. On peut noter qu'une forme allongée de la cavité peut contribuer à donner une direction préférentielle aux molécules injectées. La cavité peut être fermée ou ouverte et sa forme est adaptée au polymère ou éventuellement au cristal liquide qui forme le matériau actif.

Une étape ultérieure non représentée peut consister en la passivation de la cellule, par exemple par une colle époxy ou d'un matériau inorganique inerte tel que de la silice déposée par évaporation.

Selon des variantes de mise en oeuvre du procédé, l'ordre des différentes étapes peut être modifié, par exemple les électrodes peuvent être réalisées après la formation de la cavité, c'est-à-dire après l'élimination du matériau sacrificiel. Toutefois, il est préférable de réaliser toutes les opérations susceptibles d'altérer le matériau actif avant le remplissage de la cavité avec ce dernier.

Finalement grâce à l'invention on obtient une cellule électro-optique facile à réaliser, permettant l'usage de matériaux actifs fragiles et présentant l'avantage de nécessiter une tension de commande de modulateur réduite.

### LISTE DES DOCUMENTS CITES DANS LA PRESENTE DEMANDE

***(1)***
   "A new electro-optic modulator based on nonlinear polymers and silicon nitride working in transverse electrical configuration" FADERL I., VALETTE S., GIDON P. REVOL F. LETI (CEA-Technologies Avancées) 08-11/11/93, Int. Conf. Hybrid Materials, Bierville (F)
***(2)***
   "A fabrication process for the integration and passivation of an electro-optic polymer in an integrated optics circuit" FADERL I, LABEYE P, GIDON P, MOTTIER P LETI (CEA-Technologies Avancées) 09-13/01/94, ICONO 1, Val Thorens (F)
***(3)***
   "Polarization-independent integrated electro-optic phasemodulator in polymers" A. BRAUER, T. GASE, L. ERDMANN, P. DANNBERG, W. KARTHE Fraunhofer-Institution for Applied Optics and Precision Mechanics, Conférence SPIE 2042 "Optics Quebec 93".
***(4)***
   "20GHz electro-optic polymer Mach-Zehnder modulator" D.G. GIRTON, S.L. KWIATKOWSKI, G.F. LIPSCOMB et R.S. LYTEL Appl. Phys. Lett. 58, 22 April 1991, pages 1730-1732.
***(5)***
   US-A-4 887 884
***(6)***
   "Traveling-wave polymeric optical intensity modulator with more than 40GHz of 3dB electrical bandwidth" C.C. TENG Appl. Phys. Lett. 60, 30 March 1992, pages 1538-1540.

## Revendications

1. Cellule électro-optique à mode transverse électrique pour un modulateur comportant au moins un guide optique (6) avec une région active (10) en un matériau (12) non-linéaire ayant un indice de réfraction pouvant être modifié sous l'application d'un champ électrique appliqué au matériau, et des électrodes de commande (16) pour l'application du champ électrique disposées de part et d'autre du guide (6), caractérisée en ce qu'elle comporte en outre des régions (14) en un matériau de confinement disposées respectivement de part et d'autre et contre la région active (10), et entre les électrodes de commande (16), le matériau de confinement présentant un indice de réfraction inférieur à celui du matériau de la région active, et une résistivité électrique inférieure à celle du matériau de la région active.

2. Cellule électro-optique selon la revendication 1, caractérisée en ce qu'elle comporte une couche de recouvrement (22) de la région active (10) en un matériau présentant un indice de réfraction inférieur à celui du matériau de la région active.

3. Cellule électro-optique selon la revendication 2, caractérisée en ce que le matériau de la couche de recouvrement (22) est choisi parmi la silice dopée et un matériau polymère de marque Téflon AF.

4. Cellule électro-optique selon la revendication 1, caractérisée en ce que le matériau (12) de la région active (10) est un polymère non-linéaire.

5. Cellule électro-optique selon la revendication 1, caractérisée en ce que le matériau de confinement est de la silice dopée à l'azote, au phosphore ou bore ou encore au fluor.

6. Cellule électro-optique selon la revendication 1, caractérisée en ce que le guide optique (6) comporte un ruban (8) en un matériau présentant un indice de réfraction supérieur à celui du matériau (12) de la région active (10), et s'étendant le long du guide.

7. Cellule électro-optique selon la revendication 1, caractérisée en ce qu'elle comporte un substrat de support de la région active, des électrodes et du matériau de confinement, le substrat présentant une partie gravée contenant partiellement la région active.

8. Procédé de fabrication d'une cellule électro-optique selon l'une quelconque des revendications précédentes, caractérisé en ce qu'il comporte les opérations suivantes :
- formation d'un substrat (4) en un matériau isolant électrique et ayant un indice inférieur à l'indice du matériau de la couche active,
- formation sur le substrat (4) des électrodes de commande (16),
- formation des régions (14) en un matériau de confinement,
- formation d'une couche (32) de matériau sacrificiel et configuration de la couche selon un motif permettant de fixer l'emplacement et la forme de la région active,
- formation d'une couche de recouvrement (22) sur la couche de matériau sacrificiel,
- réalisation d'au moins une ouverture (24) dans la couche de recouvrement jusqu'à la couche (32) de matériau sacrificiel,
- élimination de la couche (32) de matériau sacrificiel pour former une cavité correspondant à la région active (10) et remplissage de la cavité avec un matériau non linéaire (12).

9. Procédé selon la revendication 8, caractérisé en ce que les électrodes de commande (16) sont formées postérieurement à l'élimination du matériau sacrificiel (32).

10. Procédé selon la revendication 8, caractérisé en ce que la formation des électrodes de commande (16) comporte le dépôt d'une couche d'accrochage (18) en des emplacements pour les électrodes de commande puis un dépôt de métal (19) sur la couche d'accrochage par croissance électrolytique.

11. Procédé selon la revendication 10, caractérisé en ce que le substrat est gravé pour former des dépression (28) correspondant aux emplacements des électrodes (16).

12. Procédé selon la revendication 8, caractérisé en ce qu'il comporte en outre la formation sur le substrat (4) d'un ruban (8) en un matériau d'un indice de réfraction supérieur à celui du matériau non linéaire et s'étendant longitudinalement dans la région active (10).
